# EUROPEAN PATENT SPECIFICATION

(11) **EP 1 643 845 B1**
(45) Date of publication and mention of the grant of the patent: **02.11.2011**
(21) Application number: 04740692.1
(22) Date of filing: 06.07.2004
(51) Int. Cl.: A23C 9/20, A23J 3/32, A23K 1/00, A23K 1/16, A23L 1/30

(54) **POWDEROUS FORMULATIONS OF FAT-SOLUBLE ACTIVE INGREDIENTS**
PULVERFÖRMIGE ZUSAMMENSETZUNGEN VON FETTLÖSLICHEN SUBSTANZEN
PREPARATIONS PULVERULENTES D'INGREDIENTS ACTIFS LIPOSOLUBLES

(30) Priority: 15.07.2003 EP 03016054
(43) Date of publication of application: 12.04.2006
(73) Proprietor: DSM IP Assets B.V., 6411 TE Heerlen (NL)
(72) Inventor: FUNDA, Elger, 79639 Grenzach-Wyhlen (DE); HUBER, Torsten, 4312 Magden (CH)
(74) Representative: Schwander, Kuno
(86) International application number: PCT/EP2004/007367
(87) International publication number: WO 2005/013708

(56) References cited:
- EP-A- 0 603 726
- EP-A- 0 982 038
- EP-A- 1 064 853
- GB-A- 849 787
- US-A- 2 897 118
- US-A- 4 395 422
- US-A- 5 153 177
- US-A- 5 853 761
- DATABASE WPI Section Ch, Week 198815 Derwent Publications Ltd., London, GB; Class D13, AN 1988-103895 XP002301478 & SU 1 335 243 A (A MED FOOD SUPPLY) 7 September 1987 (1987-09-07)

## Description

The present invention is concerned with novel stable powderous formulations comprising a fat-soluble active ingredient, and a process for their preparation. The novel compositions of this invention can be used as additives for food, beverages, animal feeds, cosmetics or drugs to incorporate said fat-soluble ingredients into such application forms.

US2897118 discloses highly stable vitamin A-active compositions which are prepared by reacting together formaldehyde and partially hydrolyzed casein in the presence of vitamin A ester.

WO0174175A discloses a powder containing an oxygen sensitive oil obtained by drying an emulsion of the oil, wherein the oil is encapsulated within a film forming protein which, prior to drying to form the powder, has been heated in solution, in the presence of a carbohydrate, for a time to provide sufficient Maillard reaction products to provide resistance to oxidation.

US5143737 discloses a method for the modification of a food for a ruminant mammal such that the mammal will produce a modified milk fat or meat fat which method comprises: (a) producing an emulsion of (i) a non-toxic food substance to be encapsulated; and (ii) an acid sensitive nontoxic crosslinkable material which will surround and encapsulate the substance with the proviso that no added crosslinking chemical is present in the emulsion; (b) subjecting the emulsion to reaction conditions which crosslink the crosslinkable material and encapsulate the substance.

More specifically, the present invention is concerned with stable powderous formulations comprising a fat-soluble active ingredient in a matrix of a milk protein composition, wherein the milk protein composition additionally contains a plant protein, whereby either the milk protein is partially hydrolyzed or the plant protein is a plant protein hydrolysate or whereby the milk protein is partially hydrolyzed and the plant protein is a plant protein hydrolysate, and wherein the protein is thermal cross-linked with a reducing sugar or a reducing sugar derivative, wherein the cross-linking of the protein is achieved by submitting the dry powder to heat treatment.

As used herein, the term "milk protein" denotes any native protein found in milk, particularly cow milk, and partially hydrolyzed proteins obtained therefrom, such as caseinates and hydrolyzed or partially hydrolyzed caseinates, lactoglobulines, whey proteins or milk powder and their hydrolyzates which can be obtained by known methods (e.g., by a acidic or alcaline treatment of the protein, or by enzymatic treatment with a protease) with degree of hydrolysis (DH) of, e.g., up to 25%, up to 15%, or up to 10%. Especially preferred are caseinates such as sodium caseinates, and hydrolyzates or partially hydrolyzed caseinates, whey protein isolates or hydrolyzed whey proteins having a protein content of more than 80 % by weight and degree of hydrolysis of up to 25%, up to 15%, or up to 10%.

In a particular aspect of the invention, a plant protein hydrolysate is used at least 80 % of which has a molecular weight distribution below 2500 Daltons. Such proteins may be present in the formulations of the invention in an amount of from 2- 20 wt.-% based on the total amount of protein in the dry formulation. The use of mixtures with plant proteins or plant protein hydrolysates may reduce loss of active ingredient in thermal treatment of the formulation and may improve stability of active ingredient. The term "milk protein composition" thus comprises milk protein and/or partially hydrolyzed milk proteins as well as mixtures thereof with plant proteins or hydrolyzed plant proteins.

The term "fat-soluble active ingredient" as used herein denotes any physiologically active ingredient that is soluble in lipids and insoluble or sparingly soluble in water. Examples of such fat-soluble active ingredients are fat-soluble vitamins, viz., vitamin A, D, E and K and derivatives thereof such as vitamin A esters, e.g. vitamin A acetate and palmitate, and vitamin E esters, e.g. tocopherol acetate; carotenoids and carotinoid derivatives, e.g., are α-or β-carotene, 8'-apo-β-carotenal, 8'-apo-β-carotenoic acid esters such as the ethyl ester, canthaxanthin, astaxanthin, astaxanthin esters, lycopene, lutein, zeaxanthin or crocetin and their derivatives; polyunsaturated fatty acids, e.g. eicosapentaenoic acid, docosahexaenoic acid, arachidonic acid and and γ-linolenic acid and/or ethylester. Such fat-soluble ingredients can be present in the formulations according to the present invention in an amount of up to 55 % by weight.

In another aspect of the invention, the novel formulations additionally contain a reducing sugar, e.g. glucose, fructose, saccharose or xylose, or a reducing sugar derivative, e.g., a desoxy sugar such as 2-desoxy-D-ribose or rhamonose, or an amino sugar e.g., a glucosamine such as 2-amino-2-desoxy-D-glucose. The sugar can be present in an amount of up to 15 % by weight, preferably, 5 -15 % by weight, especially 5 -9 % by weight, based on the dry mass of the formulation. Such formulations can be submitted to heat-treatment to cause cross-linking of the sugar with the protein in a Maillard type reaction. Crosslinking can be also achieved by treatment with enzymes like transglutaminase. The cross-linked formulations have been found to exhibit increased stability.

Particular aspects of the present invention which is defined by the claims, relate to formulations wherein the milk protein composition is :
A caseinate, especially sodium caseinate;
a mixture of caseinate, especially sodium caseinate, and a reducing sugar, e.g. fructose;
a mixture of caseinate, especiall sodium caseinate, a hydrolyzed plant protein, especially rice or soy or potato protein, and a reducing sugar, e.g. fructose;
a mixture of caseinate, especially sodium caseinate, and a carbohydrate or carbohydrate derivative and/or hydrolyzed plant protein, especially hydrolyzed rice or soy or potato protein, and a reducing sugar, e.g. fructose;
a mixture of hydrolyzed caseinate, especially hydrolyzed sodium caseinate, and a reducing sugar, e.g. fructose;
a mixture of hydrolyzed caseinate, especiall hydrolized sodium caseinate, a hydrolyzed plant protein, especially rice or soy or potato protein, and a reducing sugar, e.g. fructose;
a mixture of hydrolyzed caseinate, especially hydrolyzed sodium caseinate, and a carbohydrate or carbohydrate derivative and/or hydrolyzed plant protein, especially hydrolyzed rice or soy or potato protein, and a reducing sugar, e.g. saccharose.

In accordance with the invention, the novel formulations can be obtained by a process which comprises preparing an aqueous emulsion of the fat-soluble active ingredient and the milk protein composition, if desired, adding a reducing sugar, converting the emulsion into a dry powder and, if required, submitting the dry powder to cross-linking the sugar with the protein by heat treatment or cross-linking the protein by treatment with a cross-linking enzyme.

Suitably, in a first step of the process of the invention, the milk protein composition is dispersed in water. Thereafter, the fat-soluble active ingredient is emulsified, suitably in liquid state, i.e. with adequate warming and/or as a solution in an appropriate solvent, into the aqueous dispersion of the protein. Alternatively a suspension of the solid active may be produced by appropriate procedures like milling. The emulsion is then, optionally after removal of excess solvent, sprayed. The spraying can effected be using conventional technology of spray-drying, spray drying in combination with fluidized-bed granulation (the latter technique commonly known as fluidized spray drying or FSD), or by a powder-catch technique where sprayed emulsion droplets are caught in a bed of an absorbant such as starch or calcium silicate or silicic acid or calcium carbonate or mixtures thereof and subsequently dried.

Finally, in a still further aspect, the present invention is concerned with food, beverages, animal feeds, cosmetics and drugs which comprise the novel formulations of the present invention.

The novel formulations of this invention may further contain adjuvants and/or excipients such as one or more of a mono- di-, oligo- or polysaccharide, a triglyceride, a water-soluble antioxidant, a fat-soluble antioxidant, humectants such as glycerol, sorbitol, polyethylene glycol, propylene glycol, extenders and solubilizers., silicic acid, Ca-silicate, Ca-carbonate and water.

Examples of mono- and disaccharides which may further be present in the formulations of the present invention are saccharose, invert sugar, glucose, fructose, xylose, lactose and maltose. Examples of oligo- or polysaccharides which may further be present in the compositions of the present invention are xanthan gum, acacia gum, pectins, guar, caroub gums, alginates, celluloses, cellulose derivatives like carboxymethylcellulose, starch, modified starch and starch hydrolysates, such as dextrins and maltodextrins, especially such in the range of 5-65 dextrose equivalents (hereinafter: DE) and glucose syrup, especially such in the range of 20-95 DE. The term "dextrose equivalent" (DE) denotes the degree of hydrolysation and is measure for the amount of reducing sugar calculated as D-glucose based on dry weight. Native starch has DE close to 0 while glucose has a DE = 100.

The triglyceride is suitably a vegetable oil or fat, such as corn oil, sunflower oil, soybean oil, safflower oil, rape seed oil, peanut oil, arachis oil, palm oil, palm kernel oil, cotton seed oil or cocos oil.

The water-soluble antioxidant may be ascorbic acid and salts thereof, e.g., sodium ascorbate, and the like. The fat-soluble antioxidant may be a tocopherol, e.g., dl-α-tocopherol (i.e., synthetic tocopherol), d-α-tocopherol (i.e., natural tocopherol), β- and γ-tocopherol and mixtures thereof; ascorbic acid esters of fatty acids such as ascorbyl palmitate or stearate; butyl hydroxy toluene (BHT); butyl hydroxy anisol (BHA); propyl gallate; or t-butyl hydroxy quinoline; or 6-ethoxy-1,2-dihydroxy-2,2,4-trimethylquinoline (EMQ).

The following Examples illustrate the invention further.

### Example 1 (Prior Art Example)

75 g of sodium caseinate were added to 300 ml of water and 13.2 g of glycerol. The mixture was warmed to 60 °C until dissolution occurred. To this solution, 15.1 g of sugar fructose were added and the pH of the solution was adjusted to 6.5 ± 0.2. Thereafter, 52.3 g of vitamin A acetate (2,1 x 10⁶ IE vitamin A /g stabilized with Ethoxyquin) were emulsified into the matrix solution whereupon the mixture was stirred for 45 minutes at 60 °C. The inner phase of the emulsion then exhibited a mean particle size of about 350 nm. The emulsion was then diluted with ca. 100 ml of water and about 300 g of the emulsion was sprayed in a spraying pan in a bed of Ca-silicate at about 5° C by means of a rotating spraying nozzle. The so-obtained beadlets werde separated from excess Ca-silicate by sieving and dried. There were obtained ca. 120 g of dry powder having a vitamin A content of ca. 750'000 IEA/g.

50 g of the so-obtained dry powder were cross-linked by thermically treating the powder at 135 °C in a rotating dryer for 30 minutes. The so-obtained product had a vitamin A content of ca. 450'000 IE per g and was insoluble in hot water.

### Example 2

72.4 g of sodium caseinate and 8 g of soy protein hydrolysate (molecular weight distribution <2000 D ≅ 90%) were added to 300 ml of water and 9.5 g of glycerol and dissolved at 60 °C. To this solution, 14.5 g of sugar fructose were added and the pH of the solution was adjusted to 6.5 ± 0.2. Thereafter, 48 g of vitamin A acetate (2,1 x 10⁶ IE vitamin A /g stabilized with Ethoxyquin) were emulsified into the matrix solution whereupon the mixture was stirred for 45 minutes at 60 °C. The inner phase of the emulsion then exhibited a mean particle size of about 380 nm. About 300 g of the emulsion was sprayed in a spraying pan in analogy to the procedure described in Example 1. There were obtained about 100 g of dry powder having a vitamin A content of ca. 735'000 IE per g.

50 g of the so-obtained dry powder were cross-linked by thermically treating the powder at 125 °C in a fluidized bed dryer for 30 minutes. The so-obtained product had a vitamin A content of ca. 605000 IE per g and was insoluble in hot water.

## Claims

1. Stable powderous formulations comprising a fat-soluble active ingredient in a matrix of a milk protein composition, wherein the milk protein composition additionally contains a plant protein,
whereby either the milk protein is partially hydrolyzed or the plant protein is a plant protein hydrolysate or whereby the milk protein is partially hydrolyzed and the plant protein is a plant protein hydrolysate, and
wherein the protein is thermal cross-linked with a reducing sugar or a reducing sugar derivative, wherein the cross-linking of the protein is achieved by submitting the dry powder to heat treatment.

2. Formulations according to claim 1, wherein the milk protein composition is a native milk protein or partially hydrolyzed milk protein with a degree of hydrolysis of up to 25% or mixtures thereof having a protein content of more than 80 wt.-%.

3. Formulations according to claim 1, wherein the milk protein composition is a native milk protein or partially hydrolyzed milk protein with a degree of hydrolysis of up 15 % or mixtures thereof having a protein content of more than 80 wt.-%.

4. Formulations according to claim 1, wherein the milk protein composition is a native milk protein or partially hydrolyzed milk protein with a degree of hydrolysis of up 10 % or mixtures thereof having a protein content of more than 80 wt.-%.

5. Formulations according to any one of claims 1 to 4, wherein the milk protein is a caseinate or partially hydrolyzed caseinate.

6. Formulations according to claim 1, wherein the milk protein is a caseinate and the plant protein is a plant protein hydrolysate.

7. Formulations according to claim 1, wherein the milk protein is a partially hydrolyzed caseinate and the plant protein is a plant protein or a plant protein hydrolysate.

8. Formulations according to claim 6 or 7 wherein the average molecular weight of at least 80 % of the plant protein hydrolysate is below 2500 Daltons.

9. Formulations according to any one of claims 1 to 8, wherein the plant protein or plant protein hydrolysate is obtained from potato protein, soy protein, wheat protein, pea protein, rice protein or lupin protein.

10. Formulations according to any one of claims 1 - 9, wherein the milk protein composition contains additionally a carbohydrate or carbohydrate derivative, e.g. saccharose, invert sugar, glucose, fructose, xylose, lactose, maltose, xanthan gum, acacia gum, pectins, guar, caroub gums, alginates, celluloses, cellulose derivatives, starch, modified starch and starch hydrolysates, such as dextrins and maltodextrins, especially such in the range of 5-65 dextrose equivalents (hereinafter: DE) and glucose syrup, especially such in the range of 20-95 DE.

11. Formulations according to any one of claims 1 -10 further comprising an adjuvant.

12. Formulations according to claim 11 wherein the adjuvant is calcium silicate, silicic acid, starch or calcium carbonate, or mixture thereof.

13. Formulations according to any one of claims 1 - 12, wherein the fat-soluble active ingredient is vitamin A, D, E or K, or a carotenoid, or a polyunsaturated fatty acid, or esters thereof,or mixtures thereof.

14. Formulations according to claim 13, wherein the fat-soluble active ingredient is mixed with a plant or animal oil or fat, e.g. sunflower oil, palm oil or corn oil.

15. Formulations according to any one of claims 1 - 14, wherein the reducing sugar is glucose, fructose, saccharose or xylose.

16. Stable powderous formulations according to claim 1 or 7, wherein the milk protein is a partially hydrolyzed milk protein with a degree of hydrolysis of 3.5% to 25%.

17. Food, beverages, animal feeds, cosmetics or drugs comprising a formulation according to any one of claims 1 - 16.

18. Process for the preparation of formulations according to any one of claims 1-16, which comprises preparing an aqueous emulsion of the fat-soluble active ingredient and the milk protein composition, adding a reducing sugar or a reducing sugar derivative, converting the emulsion into a dry powder, and submitting the dry powder to cross-linking the protein by heat treatment.

## Patentansprüche

1. Stabile pulverförmige Zusammensetzungen, umfassend einen fettlöslichen Wirkstoff in einer Matrix aus einer Milchproteinzusammensetzung, wobei die Milchproteinzusammensetzung zusätzlich ein Pflanzenprotein enthält,
wobei entweder das Milchprotein partiell hydrolysiert ist oder es sich bei dem Pflanzenprotein um ein Pflanzenproteinhydrolysat handelt oder wobei das Milchprotein partiell hydrolysiert ist und es sich bei dem Pflanzenprotein um ein Pflanzenproteinhydrolysat handelt, und
wobei das Protein thermal mit einem reduzierenden Zucker oder einem Derivat eines reduzierenden Zuckers vernetzt ist, wobei das Vernetzen des Proteins dadurch erzielt wird, dass man das Trockenpulver einer Hitzebehandlung unterzieht.

2. Formulierungen nach Anspruch 1, wobei es sich bei dem Milchprotein um ein natives Milchprotein oder ein partiell hydrolysiertes Milchprotein mit einem Hydrolysegrad von bis zu 25% oder um Mischungen davon mit einem Proteingehalt von mehr als 80 Gew.-% handelt.

3. Formulierungen nach Anspruch 1, wobei es sich bei dem Milchprotein um ein natives Milchprotein oder ein partiell hydrolysiertes Milchprotein mit einem Hydrolysegrad von bis zu 15% oder um Mischungen davon mit einem Proteingehalt von mehr als 80 Gew.-% handelt.

4. Formulierungen nach Anspruch 1, wobei es sich bei der Milchproteinzusammensetzung um ein natives Milchprotein oder ein partiell hydrolysiertes Milchprotein mit einem Hydrolysegrad von bis zu 10% oder um Mischungen davon mit einem Proteingehalt von mehr als 80 Gew.-% handelt.

5. Formulierungen nach einem der Ansprüche 1 bis 4, wobei es sich bei dem Milchprotein um ein Caseinat oder ein partiell hydrolysiertes Caseinat handelt.

6. Formulierungen nach Anspruch 1, wobei es sich bei dem Milchprotein um ein Caseinat und bei dem Pflanzenprotein um ein Pflanzenproteinhydrolysat handelt.

7. Formulierungen nach Anspruch 1, wobei es sich bei dem Milchprotein um ein partiell hydrolysiertes Caseinat und bei dem Pflanzenprotein um ein Pflanzenprotein oder ein Pflanzenproteinhydrolysat handelt.

8. Formulierungen nach Anspruch 6 oder 7, wobei das durchschnittliche Molekulargewicht von mindestens 80% des Pflanzenproteinhydrolysats unter 2500 Dalton liegt.

9. Formulierungen nach einem der Ansprüche 1 bis 8, wobei das Pflanzenprotein oder Pflanzenproteinhydrolysat aus Kartoffelprotein, Sojaprotein, Weizenprotein, Erbsenprotein, Reisprotein oder Lupinenprotein stammt.

10. Formulierungen nach einem der Ansprüche 1-9, wobei die Milchproteinzusammensetzung zusätzlich ein Kohlenhydrat oder Kohlenhydratderivat enthält, z.B. Saccharose, Invertzucker, Glucose, Fructose, Xylose, Lactose, Maltose, Xanthangummi, Akaziengummi, Pectine, Guar, Carobgummen, Alginate, Zellulosen, Zellulosederivate, Stärke, modifizierte Stärke und Stärkehydrolysate, wie Dextrine und Maltodextrine, insbesondere im Bereich von 5-65 Dextroseäquivalenten (im Folgenden DE) sowie Glucosesirup, insbesondere im Bereich von 20-95 DE.

11. Formulierungen nach einem der Ansprüche 1-10, die weiterhin einen Hilfsstoff umfassen.

12. Formulierungen nach Anspruch 11, wobei es sich bei dem Hilfsstoff um Calciumsilikat, Kieselsäure, Stärke oder Calciumcarbonat oder einer Mischung davon handelt.

13. Formulierungen nach einem der Ansprüche 1-12, wobei es sich bei dem fettlöslichen Wirkstoff um Vitamin A, D, E oder K oder um ein Carotinoid oder um eine mehrfach ungesättigte Fettsäure, oder Ester davon, oder Mischungen davon, handelt.

14. Formulierungen nach Anspruch 13, wobei der fettlösliche Wirkstoff mit einem pflanzlichen oder tierischen Öl oder Fett, z.B. Sonnenblumenöl, Palmöl oder Maiskeimöl, gemischt ist.

15. Formulierungen nach einem der Ansprüche 1-14, wobei es sich bei dem reduzierenden Zucker um Glucose, Fructose, Saccharose oder Xylose handelt.

16. Stabile pulverförmige Formulierungen nach Anspruch 1 oder 7, wobei es sich bei dem Milchprotein um ein partiell hydrolysiertes Milchprotein mit einem Hydrolysegrad von 3, 5% bis 25% handelt.

17. Nahrungsmittel, Getränke, Tierfuttermittel, Kosmetika oder Arzneimittel, umfassend eine Formulierung nach einem der Ansprüche 1-16.

18. Verfahren zur Herstellung von Formulierungen nach einem der Ansprüche 1 bis 16, das Folgendes umfasst: Herstellen einer wässrigen Emulsion des fettlöslichen Wirkstoffs und der Milchproteinzusammensetzung, Versetzen mit einem reduzierenden Zucker oder einem Derivat eines reduzierenden Zuckers, Überführen der Emulsion in ein Trockenpulver, und Unterziehen des Trockenpulvers einer Proteinvernetzung mittels Hitzebehandlung.

## Revendications

1. Formulations pulvérulentes stables comprenant un ingrédient actif liposoluble dans une matrice d'une composition de protéine du lait, la composition de protéine du lait contenant en outre une protéine végétale,
ce par quoi soit la protéine du lait est partiellement hydrolysée soit la protéine végétale est un hydrolysat de protéine végétale ou ce par quoi la protéine du lait est partiellement hydrolysée et la protéine végétale est un hydrolysat de protéine végétale, et
la protéine étant réticulée par voie thermique avec un sucre réducteur ou un dérivé de sucre réducteur, la réticulation de la protéine étant réalisée en soumettant la poudre sèche à un traitement par la chaleur.

2. Formulations selon la revendication 1, **caractérisées en ce que** la composition de protéine du lait est une protéine du lait native ou une protéine du lait partiellement hydrolysée ayant un degré d'hydrolyse allant jusqu'à 25 % ou des mélanges de celles-ci ayant une teneur en protéine supérieure à 80 % en poids.

3. Formulations selon la revendication 1, **caractérisées en ce que** la composition de protéine du lait est une protéine du lait native ou une protéine du lait partiellement hydrolysée ayant un degré d'hydrolyse allant jusqu'à 15 % ou des mélanges de celles-ci ayant une teneur en protéine supérieure à 80 % en poids.

4. Formulations selon la revendication 1, **caractérisées en ce que** la composition de protéine du lait est une protéine du lait native ou une protéine du lait partiellement hydrolysée ayant un degré d'hydrolyse allant jusqu'à 10 % ou des mélanges de celles-ci ayant une teneur en protéine supérieure à 80 % en poids.

5. Formulations selon l'une quelconque des revendications 1 à 4, **caractérisées en ce que** la protéine du lait est un caséinate ou un caséinate partiellement hydrolysé.

6. Formulations selon la revendication 1, **caractérisées en ce que** la protéine du lait est un caséinate et la protéine végétale est un hydrolysat de protéine végétale.

7. Formulations selon la revendication 1, **caractérisées en ce que** la protéine du lait est un caséinate partiellement hydrolysé et la protéine végétale est une protéine végétale ou un hydrolysat de protéine végétale.

8. Formulations selon la revendication 6 ou 7, **caractérisées en ce que** le poids moléculaire moyen d'au moins 80 % de l'hydrolysat de protéine végétale est inférieur à 2500 Daltons.

9. Formulations selon l'une quelconque des revendications 1 à 8, **caractérisées en ce que** la protéine végétale ou l'hydrolysat de protéine végétale est obtenu à partir de la protéine de pomme de terre, de la protéine de soja, de la protéine de blé, de la protéine de pois, de la protéine de riz ou de la protéine de lupin.

10. Formulations selon l'une quelconque des revendications 1 à 9, **caractérisées en ce que** la composition de protéine du lait contient en outre un glucide ou un dérivé de glucide, par exemple le saccharose, le sucre inverti, le glucose, le fructose, le xylose, le lactose, le maltose, la gomme xanthane, la gomme acacia, les pectines, le guar, les gommes de caroube, les alginates, les celluloses, les dérivés de cellulose, l'amidon, l'amidon modifié et les hydrolysats d'amidon, tels que les dextrines et les maltodextrines, notamment par exemple dans la gamme de dextrose équivalent (ci-après : DE) 5-65, et le sirop de glucose, notamment par exemple dans la gamme de DE 20-95.

11. Formulations selon l'une quelconque des revendications 1 à 10, comprenant en outre un adjuvant.

12. Formulations selon la revendication 11, **caractérisées en ce que** l'adjuvant est le silicate de calcium, l'acide silicique, l'amidon ou le carbonate de calcium, ou des mélanges de ceux-ci.

13. Formulations selon l'une quelconque des revendications 1 à 12, **caractérisées en ce que** l'ingrédient actif liposoluble est la vitamine A, D, E ou K, ou un caroténoïde, ou un acide gras polyinsaturé, ou des esters de celui-ci, ou des mélange de ceux-ci.

14. Formulations selon la revendication 13, **caractérisées en ce que** l'ingrédient actif liposoluble est mélangé avec une huile ou graisse végétale ou animale, par exemple l'huile de tournesol, l'huile de palme ou l'huile de maïs.

15. Formulations selon l'une quelconque des revendications 1 à 14, **caractérisées en ce que** le sucre réducteur est le glucose, le fructose, le saccharose ou le xylose.

16. Formulations pulvérulentes stables selon la revendication 1 ou 7, **caractérisées en ce que** la protéine du lait est une protéine du lait partiellement hydrolysée ayant un degré d'hydrolyse de 3,5 % à 25 %.

17. Aliment, boissons, aliments pour animaux, produits cosmétiques ou médicaments comprenant une formulation selon l'une quelconque des revendications 1 à 16.

18. Procédé de préparation de formulations selon l'une quelconque des revendications 1 à 16, **caractérisé en ce qu'**il consiste à préparer une émulsion aqueuse de l'ingrédient actif liposoluble et de la composition de protéine du lait, ajouter un sucre réducteur ou un dérivé de sucre réducteur, transformer l'émulsion en une poudre sèche, et soumettre la poudre sèche à une réticulation de la protéine par traitement par la chaleur.
